# EUROPEAN PATENT APPLICATION

(11) **EP 3 130 623 A1**
(43) Date of publication of application: **15.02.2017**
(21) Application number: 16166900.7
(22) Date of filing: 08.02.2010
(51) Int. Cl.: C08G 63/183, C08L 67/03, B65D 65/46, D01F 6/62, C08L 101/16

(54) **BIO-BASED POLYETHYLENE TEREPHTHALATE PACKAGING AND METHOD OF MAKING THEREOF**

(30) Priority: 03.03.2009 WO PCT/US2009/035849; 12.10.2009 US 577480
(62) Divisional of application: 10749089.8
(71) Applicant: The Coca-Cola Company, Atlanta, GA 30313 (US)
(72) Inventor: KRIEGEL, Robert M., GA 30030 Decatur (US); HUANG, Xiaoyan, GA 30068 Marietta (US); SCHULTHEIS, Mikell, GA 30102 Acworth (US); KOLLS, Brock, H., GA 30005 Alpharetta (US)
(74) Representative: Towler, Philip Dean

(57) **Abstract**

This invention relates to a method of making a bio-based polyethylene terephthalate (PET) container comprising: (a) forming monoethylene glycol (MEG) from at least one bio-based material; (b) melt polymerizing the MEG with terephthalic acid (TA) to form a bio-based PET; (c) solid state polymerizing the bio-based PET to form a PET resin; and (d) processing the PET resin into a PET container.

## Description

### RELATED APPLICATION DATA

The present application is a continuation-in-part of U.S. non-provisional application Ser. No. 12/210,208, entitled "Bio-based Polyethylene Terephthalate and Articles Made from Bio-based Polyethylene Terephthalate" and filed on September 14, 2008, which claims priority under 35 U.S.C. §119(e) to U.S. Provisional Application Number 61/040349 of the same title, filed on March 28, 2008. The aforementioned disclosures are hereby incorporated by reference in their entirety for all purposes.

### TECHNICAL FIELD OF THE INVENTION

This invention relates to a method of making bio-based PET packaging and particularly to a method of making and recycling bio-based PET packaging.

### BACKGROUND OF THE INVENTION

As used herein, the term "PET" refers to polyethylene terephthalate, its copolyesters, and combinations thereof in any form including PET flakes, pellets and recycled PET. The term "PET products" refers to products made from PET, including but not limited to resins, performs, and PET packaging. The term "PET packaging" as used herein shall refer to all PET packaging including but not limited to PET packaging used for packaging food products, soft drinks, alcoholic beverages, detergents, cosmetics, pharmaceutical products and edible oils such as PET containers (which encompasses bottles) and PET secondary packaging, which is usually used for organizing and securing for transport, display, and storage of PET containers as well as for advertising the product contained within.

The term "bio-based," as used herein, indicates the inclusion of some component that partially or totally derives from at least one bio-based material. As an example, a "bio-based PET" would be a PET that comprises at least one component that partially or totally derives from at least one bio-based material. The term "bio-based materials" and "renewable materials" both refer to organic materials in which the carbon comes from non-fossil biological sources.

PET is a widely used raw composition for making packaging articles in part due to their excellent combination of clarity, mechanical, and gas barrier properties. Today, most commercial methods produce PET with petrochemically derived raw materials (hereinafter referred to as "petroleum-based PET"). Therefore, the cost of production is closely tied to the price of petroleum. Petroleum-based PET contributes to greenhouse emissions due to its high petroleum derived carbon content. Furthermore, petrochemicals take hundreds of thousands of years to form naturally, making petrochemically-derived products non-renewable, which means they cannot be re-made, re-grown, or regenerated at a rate comparable to its consumption.

As regulations become more rigorous with regard to the environmental impact of industrial activities and as petroleum resources become increasingly scarce, there exists a growing need for a bio-based PET that may serve as an alternative to petroleum-based PET. It would be further desirable if the bio-based PET has similar chemical and/or physical properties and/or chemical structures as petroleum-based PET so that technology and facilities currently designed for petroleum-based PET can be readily applied to bio-based PET. For example, in some applications, it would be desirable if bio-based PET products may be processed through existing petroleum-based PET product manufacturing facilities and/or readily recycled through the systems designed for recycling petroleum-based PET products.

Bio-based materials would also satisfy consumers' increasing demand of products that are environmentally friendly. It would be more desirable if the bio-based materials do not compete with foods or food-grade materials that may potentially increase the costs of necessity items for consumers. For example, the bio-based materials may be obtained from a food or agricultural waste stream. Thus, there is a need to produce PETs derived from bio-based materials that do not compete with foods or food-grade materials.

Other objects, features, and advantages of this invention will be apparent from the following detailed description, drawings, and claims.

### BRIEF DESCRIPTION OF THE FIGURES

FIG. 1 is a flowchart illustration of an embodiment of making a bio-based PET container from sugarcane.
FIG. 2 is a flowchart illustration of an embodiment of making a bio-based PET container from citrus.
FIG. 3 is a flowchart illustration of an embodiment of recycling a bio-based PET container.

The detailed description explains the embodiments of the invention, together with advantages and features, by way of example with reference to the drawings.

### DETAILED DESCRIPTION OF THE INVENTION

Embodiments of the present invention encompass a bio-based PET comprising at least one bio-based material. Alternatively, the bio-based PET may be processed into a composition selected from a PET resin, a PET fiber, a PET packaging, and combinations thereof. In particular embodiments, the bio-based material may be selected from sugars, starches, corns, natural fibers, sugarcanes, beets, citrus fruits, woody plants, cellulosics, lignocelluosics, hemicelluloses, potatoes, plant oils, oily wood feedstock, other polysaccharides such as pectin, chitin, levan, pullulan, and combinations thereof. More particularly, the bio-based material may be selected from wood chips, citrus peels, forestry waste, agriculture waste, crop husks, and bio-based materials with greater than 3% in content of cellulose, hemicelluloses, lignin, and combinations thereof. The selection of bio-based materials may depend on factors including, but not limited to, supply availability, cost, type of technology used, and environmental impact.

Steps of method in the embodiments recited herein need not be performed in the order of steps described. One skilled in the art would know which steps may be performed simultaneously or chronologically and at the same or different locations.

Embodiments of the present invention also encompass methods of producing a bio-based PET from at least one bio-based material comprising the following steps: a) forming at least one PET component from at least one bio-based material, wherein the at least one PET component is selected from a monoethylene glycol ("MEG"), a terephthalic acid ("TA"), and combinations thereof; and b) processing said bio-based PET component into a bio-based PET. More particularly, the PET component may be a MEG and melt polymerized with a TA to produce a PET. Alternatively, the PET component may be a TA and melt polymerized with a MEG to produce a PET. Yet more alternatively, the PET component may be combinations of MEG and TA, which may be melt polymerized to produce a PET. More particularly, the melt polymerization step further comprises mixing the MEG and the TA in a catalyst solution, promoting esterification between the MEG and TA at atmospheric pressure to form a bio-based PET, optionally separating the impurities from the bio-based PET, and polycondensing the bio-based PET. Alternatively, the method further comprises processing the bio-based PET to form a fiber or a filament. In yet another alternative embodiment, the method further comprises solid state polymerizing the bio-based PET to form a PET resin. More particularly, the PET resin may be further processed into a PET perform, a PET packaging, and combinations thereof.

More particularly, the MEG and the TA may be produced from the bio-based material using methods including but not limited to fast pyrolysis, acid hydrolysis, enzymatic hydrolysis, microbial degradation, mycological degradation, and hydrogenolysis. Alternatively, the PET packaging may be partially or totally derived from at least one bio-based material.

Alternatively, the bio-based material is used to produce a PET packaging, wherein the PET packaging comprises an edible product. More particularly, the edible product further comprises the bio-based material. In another embodiment, the bio-based material may be used to produce an ingredient and the edible product further comprises the ingredient. In yet another embodiment, the ingredient may be selected from sugar, ethanol, carbon dioxide, and combinations thereof.

### Method of Producing Bio-Based PET: Sugarcane

Today, a typical shortcoming of sugarcane refining is that after the sugarcane has been refined into sugar and molasses, the leftover cane husks (or sometimes called bagasse) are often discarded in landfills or burned for fuel or used for animal feed. Bagasse is rich in cellulose, hemicelluloses, and lignin but has practically no food value. Finding alternative ways to use the leftover bagasse to produce a bio-based PET would reduce waste. In the following methods, sugar beets may also be used in place of sugarcane.

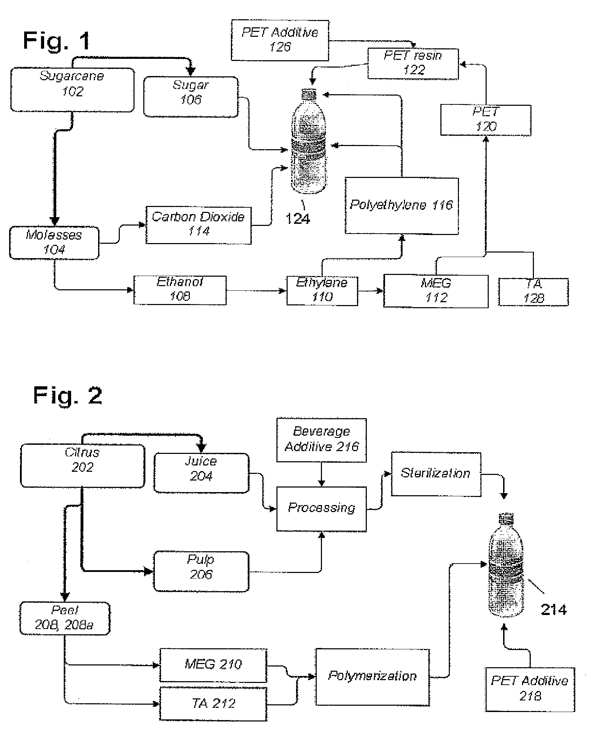

Referring to Figure 1, a particular embodiment of the present invention encompasses a method of producing a bio-based PET from sugar comprising the following steps: a) refining a sugarcane 102 into a molasses 104 and a sugar 106; b) fermenting the molasses 104 to produce an ethanol 108; c) refining the ethanol into an ethylene 110; refining the ethylene 110 into a MEG 112; and melt polymerizing the MEG 112 with a TA 128 to form a bio-based PET 120. In a more particular embodiment, the TA 128 may be a bio-based TA. In another embodiment, the method further comprises solid state polymerizing the bio-based PET to form a PET resin 122. Alternatively, the method further comprises a non solid-state polymerization step such as melt-to-resin technologies to produce the PET resin 122.

Alternatively, the PET resin 122 may be molded into a PET container 124 by various methods including but not limited to making preforms, blowing vessels, thermoforming, extrusion molding, compression molding, injection molding, extrusion blow molding and other methods. A skilled artisan would be able to determine which method is more suitable for each application considering factors including but not limited to time, cost, availability, location, design of the vessel, and function of the vessel. The PET container 124 may be used but is not limited to packaging food products, soft drinks, alcoholic beverages, detergents, cosmetics, pharmaceutical products, edible oils, and combinations thereof.

In a particular embodiment, the sugarcane 102 may be refined into the molasses 104 and the sugar 106 by crystallization and purification to produce pure sugar and residual molasses. One typical process of refining sugarcane 102 to sugar 106 and molasses 104 is by milling the sugarcane 102, mixing the sugarcane 102 with water to produce a sugar juice, heating the sugar juice to about 65°C to 70°C, mixing the sugar juice with lime and with gaseous sulfur dioxide, further heating the sugar juice to about 100°C to 105°C, precipitating the impurities, evaporating the sugar juice to create a syrup, cooling the syrup so that the sugar 106 may crystallize, and separating the sugar 106 to produce the molasses 104 (residual liquid syrup). Other refining processes may use calcium phosphate in place of lime and/or treatment activated carbon in place of sulfur dioxide for decolorization.

Alternatively, the molasses 104 may be fermented to ethanol 108 using yeast or other suitable fermentation organisms held at nutrient and temperature conditions familiar to those skilled in the art. Optionally, the method further comprises fermenting the molasses 104 to produce a carbon dioxide 114. More particularly, the carbon dioxide 114 may be captured and used to carbonate beverages stored in the PET container 124.

In one embodiment, the ethanol 108 may be refined into the ethylene 110 by dehydration with mineral acids, strong organic acids, suitable catalysts and combinations thereof. In another embodiment, the ethylene 110 may be converted to ethylene oxide by use of a catalyst and oxygen. The ethylene oxide may further be converted to MEG 112 by a reaction with water or by a reaction with acetic acid and/or carbon dioxide to produce an intermediate compound that may be hydrolyzed to MEG 112.

Alternatively, the method comprises refining the ethylene 110 into at least one polyethylene 116, wherein the polyethylene may be selected from a low-density polyethylene ("LDPE"), a high-density polyethylene ("HDPE"), a linear low density polyethylene ("LLDPE"), ultra-high molecular weight polyethylene ("UHMWPE") and combinations thereof. In a particular embodiment, the method further comprises polymerizing the ethylene 110 with a suitable catalyst under high monomer pressure and elevated temperature to produce the at least one polyethylene 116. More particularly, the at least one polyethylene 116 may be processed to form a PET packaging.

Alternatively, the method further comprises using the at least one polyethylene 116 to manufacture a closure 120 for a PET container 124. Particularly, the closure 118 may be a cap, a lid, and or other similar or suitable PET container 124 closures to be attached and or used to seal the product into the PET container 124. Alternatively, the closure 118 may be a screw type closure, snap type closure, and or other type of closure that can be used to seal and reseal the PET container 124. In another embodiment, the method further comprises using the at least one polyethylene 116 to produce a packaging label. In a more particular embodiment, the packaging label may be manufactured by extrusion of the at least one polyethylene 116 into a film of appropriate thickness and desired properties, followed by pretreatment and printing depending on applications.

Alternatively, at least one PET additive 126 may be added to the PET resin 122 and/or the PET closure 120. The PET additives 126 may be selected from colorants, ultraviolet protection additives, thermal stabilizers, reheat additives, barrier protection enhancers to improve reduction in transmission of oxygen, carbon dioxide, and or other gasses, liquids, light, or other materials through the vessel surface, and combinations thereof.

According to a particular embodiment, the PET container 124 may contain a product that comprises at least one of the sugar 106, the ethanol 108, the carbon dioxide 114, and combinations thereof produced by the methods recited above. The sugar 106, the ethanol 108, the carbon dioxide 114, and combinations thereof may be added to the product using any known industrial method such as blending, dosing, or the use of a CarboCooler^{™}. Skilled artisans would be able to determine the best method of use when considering factors including but not limited to, the type of product, the availability of equipments, cost, and manufacturing and delivery time.

In another embodiment, the PET container 124 may contain a beverage. In a more particular embodiment, the beverage comprises at least one of the sugar 106, the ethanol 108, the carbon dioxide 114, and combinations thereof produced by the methods recited above. In yet a more particular embodiment, the PET container 124 may be sealed with the PET closure 120 made from the at least one polyethylene 116.

### Method of Producing Bio-Based PET: Corn Starch

Another embodiment of the present invention encompasses a method of producing a bio-based PET from corn starch comprising the following steps: a) solubilizing the corn starch to form a starch solution or gel; b) heating the starch solution or gel in hydrogen steam catalyst to produce a mixture of glycols, wherein the mixture of glycols comprises ethylene glycol; c) purifying the mixture of glycols to form MEG; and d) melt polymerizing the MEG with the TA to form a bio-based PET. More particularly, the method further comprises solid state polymerizing the bio-based PET to form a PET resin and the PET resin may be molded into a PET container. Yet more particularly, the purification may be by distillation, crystallization, membrane separation, and combinations thereof.

### Method of Producing Bio-Based PET: Fruits, Particularly Citrus

As used herein, the term "citrus" refers to any part of a plant that produces citrus fruits including but not limited to oranges, lemons, limes, grapefruits, tangerines, any edible member of the Genus Citrus, and combinations thereof. Today, a typical shortcoming related to the citrus business is that after the juice and the pulp are extracted from the citrus, the peel is usually discarded. Finding alternative ways to use the leftover citrus peels to produce a bio-based PET would reduce waste. The same concept may be applicable to non-citrus fruits.

Referring to Figure 2, an embodiment of the present invention encompasses a method of producing a bio-based PET from a fruit comprising the following steps: a) extracting a peel 208 from a fruit 202 and b) extracting at least one peel component from said peel, wherein the at least one peel component is selected from limonene, sugar, a starch, a cellulose, and combinations thereof; c) refining the at least one peel component 208a into at least one of a MEG 210, a TA 212, and combinations thereof; and c) melt polymerizing the MEG 208 with the TA 212 to form a bio-based PET. Particularly, the method further comprises solid state polymerizing the bio-based PET to form a PET resin. More particularly, the PET resin may be molded into a PET container 214. Alternatively, the fruit is selected from oranges, lemons, limes, grapefruits, tangerines, and combinations thereof.

More particularly, the MEG 310 and the TA 312 may be produced using methods including but not limited to fast pyrolysis, acid hydrolysis, enzymatic hydrolysis, microbial degradation, mycological degradation, and hydrogenolysis.

In an alternative embodiment, the method further comprises extracting a juice 204 from the fruit 202; processing the juice 204 to form a beverage; optionally adding at least one beverage additive 216 to the beverage; sterilizing the beverage; and dispensing the beverage into the PET container 214. More particularly, the juice 204 may be processed by condensing the juice 204, debittering the juice 204, filtering the juice 204, and blending the juice 204 with at least one of other juices, flavors, colors. Yet more particularly, the juice 204 may be sterilized by pasteurization. Alternatively, the at least one beverage additive 216 may be selected from neutraceuticals, antioxidants, vitamins, minerals, and combinations thereof.

In another embodiment, the method further comprises extracting a pulp 206 from the fruit 202 and dosing the pulp 206 into the juice 204. Particularly, the pulp 206 may be selectively controlled and dosed back to the juice 204. The beverage may comprise different levels of pulp ranging from little or no pulp to extra pulp.

Particularly, the method further comprises dispensing the beverage into the PET container 214. The PET container 214 may be produced in the same or a different location from where the beverage/juice is dispensed into the PET container 214. Those skilled in the art would be able to determine the best location for production of the PET container 214 and the beverage/juice based on factors including but not limited to cost, logistics, contamination, facility capacity, and processing time.

Alternatively, at least one PET additive 218 may be added to the PET resin and/or the PET container 214. The PET additives 218 may be selected from colorants, ultraviolet protection additives, thermal stabilizers, reheat additives, barrier protection enhancers to improve reduction in transmission of oxygen, carbon dioxide, and or other gases, liquids, light, or other materials through the vessel surface, and combinations thereof.

A particular embodiment of the present invention encompasses a beverage comprising the juice of at least one fruit, wherein the juice 204 is dispensed into a bio-based PET container, wherein the bio-based PET 214 container comprises at least one of MEG 210, TA 212, and combinations thereof that derived from the peel 208 of the fruit 202. Alternatively, the juice 204 may be further processed to form a beverage. The beverage may optionally comprise at least one beverage additive 216 selected from neutraceuticals, antioxidants, vitamins, minerals, and combinations thereof.

Depending on the type of fruits, certain components including but not limited to fibers may be further processed by thermal cracking processes to produce sugars and chemicals such as para-xylene, which may be further processed to bio-based TA. When a particular fruit is chosen, a skilled artisan would readily be able to determine which components of the fruit may be processed into different components that may be used to form a bio-based PET and/or an edible product depending on available technology.

### Method of Producing Bio-Based PET: Agricultural Waste Steams

A particular embodiment encompasses a method of producing a bio-based PET from agricultural waste comprising the following steps: a) collecting an agricultural waste stream; b) refining the agricultural waste stream into a MEG; and c) melt polymerizing the MEG with a TA to form a PET. In a more particular embodiment, the TA may be a bio-based TA. In one embodiment, the method further comprises solid state polymerizing the PET to form a PET resin. More particularly, the agricultural waste stream may be selected from sugar husk, bagasse, corn stover, woodchips, other agricultural waste streams and products, and combinations thereof.

### Recycling bio-based PET Packaging

Once a bio-based PET packaging is filled with a product, the bio-based PET packaging may be distributed to a consumer through marketing outlets and other means. After the product is removed or consumed, the used bio-based PET packaging may be collected in a recycle supply chain. The recycle supply chain may include, but is not limited, one or more of the an organized array of curb side pickup, special containers available to the public in building, at events, and in other locations, using designated collection sites, and municipal recycling programs. After entering the recycle supply chain, the used bio-based PET packaging may be processed into a PET chip. The term "PET chip" as used herein refers to PET resin in the forms of chips (or sometimes referred to as pellets) and flakes that are primarily made form used PET packaging, including used bio-based PET packaging and used petroleum-based PET packaging. PET chips typically require only minimal cleaning and re-melting in order to be used in a new PET packaging.

The used bio-based PET packaging may also be processed to a recycled MEG or a recycled TA by chemical depolymerization methods such as hydrolysis, methanolysis, glycolysis, alcoholysis, aminolysis and combinations thereof.. The PET chip, the recycled MEG, and/or the recycled TA may be further processed to form new bio-based PET products. Under the industrial recycling operations available today, existing recycle supply chains are unlikely to recover a sufficient amount of used bio-based PET packaging to generate all the new PET products in demand. Thus, a supply of new bio-based MEG and new bio-based-TA will need to be continually produced to satisfy demands.

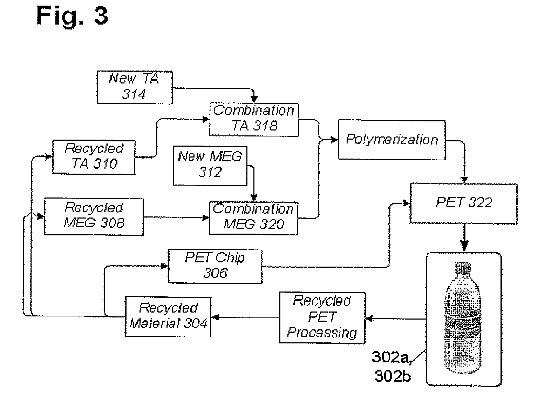

Referring to Fig. 3, a particular embodiment of the present invention encompasses a method of recycling a used bio-based PET packaging 302a comprising the steps of: a) processing a used bio-based PET 302a through a PET processing center to produce at least one recycled material 303 selected from a PET chip 306, a recycled MEG 308, a recycled TA 310, and combinations thereof. Alternatively, the method further comprises separating the at least one recycled material 303 into groups of PET chips 306, recycled MEG 308, and recycled TA 310. In one embodiment, the recycled material is a PET chip 306 and the method further comprises routing the PET chip to a molding process. Alternatively, the recycled material is a recycled MEG 308 or a recycled TA 310 and the method optionally comprises combining the recycled MEG 308 with a new bio-based MEG 312 to form a combination MEG and combining the recycled TA 310 with a new bio-based TA 313 to form a combination TA. More particularly, the method comprises polymerizing the combination MEG and the combination TA to form a new PET. Yet more particularly, the method comprises combining the recycled MEG with the new MEG at a specific ratio. Even more particularly, the method comprises combining the recycled TA with the new TA at a specific ratio. Alternatively, the new PET may be combined with the PET chip 306 to produce a PET container 302b.

Thus, a sustainable bio-based packaging may be created by utilizing both new MEG and TA made from fresh bio-based materials and recycled MEG and TA made from recycled bio-based materials.

The capabilities of the present invention can be implemented in software, firmware, hardware or combinations thereof.

As one example, one or more aspects of the present invention can be included in an article of manufacture (e.g., one or more computer program products) having, for instance, computer usable media. The media has embodied therein, for instance, computer readable program code means for providing and facilitating the capabilities of the present invention. The article of manufacture can be included as a part of a computer system or sold separately.

Additionally, at least one program storage device readable by a machine, tangibly embodying at least one program of instructions executable by the machine to perform the capabilities of the present invention can be provided.

The flow diagrams depicted herein are just examples. There may be many variations to these diagrams or the steps (or operations) described therein without departing from the spirit of the invention. For instance, the steps may be performed in a differing order, or steps may be added, deleted or modified. All of these variations are considered a part of the claimed invention.

While the preferred embodiment to the invention has been described, it will be understood that those skilled in the art, both now and in the future, may make various improvements and enhancements which fall within the scope of the claims which follow.

### Preferred Embodiments

### Embodiment 1

A method of making a bio-based polyethylene terephthalate (PET), comprising: (a) forming at least one PET component from at least one bio-based material, wherein the at least one PET component is selected from a monoethylene glycol ("MEG"), a terephthalic acid ("TA"), and combinations thereof; (b) processing said bio-based PET component into a bio-based PET.

### Embodiment 2

The method of embodiment 1, wherein the method further comprises solid state polymerizing the bio-based PET to form a PET resin and processing the PET resin into a PET preform, a PET packaging, and combinations thereof.

### Embodiment 3

The method of embodiment 2, wherein said bio-based PET packaging comprises an edible product and said edible product comprises an ingredient which is made from said bio-based material.

### Embodiment 4

The method of embodiment 1, wherein the at least one PET component is produced from the bio-based material using methods of fast pyrolysis, acid hydrolysis, enzymatic hydrolysis, microbial degradation, mycological degradation, or hydrogenolysis.

### Embodiment 5

The method of embodiment 1, wherein the method further comprises processing the bio-based PET to form a fiber or a filament.

### Embodiment 6

A method of making a bio-based polyethylene terephthalate (PET), comprising (a) refining a sugarcane into a molasses and a sugar; (b) fermenting the molasses to produce an ethanol; (c) refining the ethanol into an ethylene; and (d) refining the ethylene into a MEG, at least one polyethylene, and combinations thereof.

### Embodiment 7

The method of embodiment 6, further comprising melt polymerizing the MEG with a TA to form a bio-based PET and processing said bio-based PET to a PET container.

### Embodiment 8

The method of embodiment 7, further comprising fermenting the molasses to produce a carbon dioxide and using said carbon dioxide and said sugar as a beverage component for a beverage stored in said PET container.

### Embodiment 9

The method of embodiment 7, further comprising processing the at least one polyethylene to form a closure for said PET container.

### Embodiment 10

The method of embodiment 7, further comprising processing the at least one polyethylene to form a PET secondary packaging for said PET container.

### Embodiment 11

The method of embodiment 6, further comprising processing the at least one polyethylene into a PET packaging, wherein the at least one polyethylene is selected from a low-density polyethylene ("LDPE"), a high-density polyethylene ("HDPE"), a linear low density polyethylene ("LLDPE"), ultra-high molecular weight polyethylene ("UHMWPE") and combinations thereof.

### Embodiment 12

A method of making a bio-based polyethylene terephthalate (PET), comprising (a) solubilizing the corn starch 202 to form a starch solution; (b) heating said starch solution in hydrogen steam catalyst to produce a mixture of glycols, wherein said mixtures of glycols comprises ethylene glycol; (c) purifying said mixture of glycols to form a MEG; and (d) melt polymerizing said MEG with a TA to form a bio-based PET.

### Embodiment 13

A method of making a bio-based polyethylene terephthalate (PET), comprising: (a) extracting a peel from a fruit; (b) extracting at least one peel component from said peel, wherein the at least one peel component is selected from limonene, sugar, a starch, a cellulose, and combinations thereof; (c) refining the at least one peel component into at least one PET component, wherein the at least one PET component is selected from a MEG, a TA, and combinations thereof; and (d) melt polymerizing the at least one PET component to form a bio-based PET.

### Embodiment 14

The method of embodiment 13, wherein the fruit is selected from oranges, lemons, limes, grapefruits, tangerines, coconuts, and combinations thereof.

### Embodiment 15

The method of embodiment 13, further comprising extracting a juice from said fruit, processing said juice to form a beverage.

### Embodiment 16

The method of embodiment 15, further comprising extracting a pulp from said fruit and dosing said pulp into said juice or beverage.

### Embodiment 17

The method of embodiments 15-16, further processing said bio-based PET to form a bio-based PET container and dispensing said beverage into said PET container.

### Embodiment 18

A method of making a bio-based polyethylene terephthalate (PET), comprising (a) collecting an agricultural waste stream; (b) refining the agricultural waste stream into a MEG; and (c) melt polymerizing the MEG with a TA to form a bio-based PET.

### Embodiment 19

A method of recycling a bio-based PET packaging comprising (a) processing a bio-based PET through a PET processing center to produce at least one recycled material selected from a PET chip, a recycled MEG, a recycled TA, and combinations thereof; (b) separating the at least one recycled material into groups of PET chips, recycled MEG, and recycled TA.

### Embodiment 20

The method of embodiment 19, wherein the at least one recycled material is a recycled MEG, the method further comprising said recycled MEG with a new bio-based MEG to form a combination MEG stream.

### Embodiment 21

The method of embodiments 19-20, wherein the at least one recycled material is a recycled TA, the method further comprising said recycled TA with a new bio-based MEG to form a combination TA stream.

### Embodiment 22

The method of embodiment 21, further comprising polymerizing the combination MEG stream and the combination TA stream to form a new bio-based PET.

## Claims

1. A method of making a bio-based polyethylene terephthalate (PET) container comprising:
(a) forming monoethylene glycol (MEG) from at least one bio-based material;
(b) melt polymerizing the MEG with terephthalic acid (TA) to form a bio-based PET;
(c) solid state polymerizing the bio-based PET to form a PET resin; and
(d) processing the PET resin into a PET container.

2. The method of claim 1, wherein the at least one bio-based material is an agricultural waste stream selected from sugar husk, bagasse, corn stover, woodchips and combinations thereof.

3. The method of claim 1, wherein the at least one bio-based material is selected from sugars and corns.

4. The method of claim 1 or 2, wherein the MEG is produced from the at least one bio-based material using a method selected from fast pyrolysis, acid hydrolysis, enzymatic hydrolysis, microbial degradation, mycological degradation, or hydrogenolysis.

5. The method of claim 1, wherein the PET resin is molded into a container by a method selected from making preforms, blowing vessels, thermo forming, extrusion molding, compression molding, injection molding, and extrusion blow molding.

6. The method of claim 1, wherein the container is a bottle.

7. The method of claim 1, wherein the container is used to package soft drinks.

8. The method of claim 2, wherein the agricultural waste stream is sugar husk.

9. The method of claim 2, wherein the agricultural waste stream is bagasse.

10. The method of claim 2, wherein the agricultural waste stream is corn stover.

11. The method of claim 2, wherein the agricultural waste stream is woodchips.

12. The method of claim 3, wherein the at least one bio-based material is sugars.

13. The method of claim 3, wherein the at least one bio-based material is corns.

14. The method of claim 1, wherein the PET resin further comprises one or more additives selected from colorants, ultraviolet protection additives, thermal stabilizers, reheat additives, barrier protection enhancers and combinations thereof.

15. The method of claim 1, wherein the melt polymerization comprises mixing the MEG and TA in a catalyst solution, promoting esterification between the MEG and TA at atmospheric pressure to form a bio-based PET, and optionally separating impurities from the bio-based PET.
